# EUROPEAN PATENT APPLICATION

(11) **EP 3 184 025 A1**
(43) Date of publication of application: **28.06.2017**
(21) Application number: 16836824.9
(22) Date of filing: 23.03.2016
(51) Int. Cl.: A61B 1/00, A61B 1/06, A61B 17/94, A61B 90/30

(54) **MEDICAL SHEATH AND MEDICAL DEVICE**

(30) Priority: 20.08.2015 JP 2015162859
(71) Applicant: Olympus Corporation, Tokyo 192-8507 (JP)
(72) Inventor: FU, Yuchun, Hachioji-shi Tokyo 192-8507 (JP); IGARASHI, Makoto, Hachioji-shi Tokyo 192-8507 (JP); YAJIMA, Hiroyoshi, Hachioji-shi Tokyo 192-8507 (JP); NAMIKI, Mitsuru, Hachioji-shi Tokyo 192-8507 (JP); WATANABE, Takeshi, Hachioji-shi Tokyo 192-8507 (JP)
(74) Representative: Schicker, Silvia
(86) International application number: PCT/JP2016/059139
(87) International publication number: WO 2017/029832

(57) **Abstract**

A medical sheath is a medical sheath to be used in combination with a medical device including an insertion portion that can be inserted into a body cavity of a subject via a trocar when treatment of an examined region present in the body cavity of the subject is performed using the medical device, the medical sheath including: a sheath body formed in a shape that can be inserted to and removed from a through hole through which the insertion portion is inserted in the trocar; a channel provided in the sheath body, the channel being a conduit formed so as to have a shape that enables insertion and removal of the insertion portion; and an optical transmission section provided in the sheath body, the optical transmission section being provided for guiding at least one of illuminating light to be applied to the examined region and return light emitted from the examined region upon application of the illuminating light.

## Description

### Technical Field

The present invention relates to a medical sheath and a medical appliance, and specifically relates to a medical sheath to be used together with a medical device to be inserted into a body cavity, and a medical appliance.

### Background Art

In a medical field, when treatment of an examined region inside a body cavity is performed, for example, an optical fiber for guiding light to be applied to the examined region and/or guiding light emitted from the examined region and a medical device such as a treatment instrument to be inserted into the body cavity may be used in combination.

More specifically, for example, Japanese Patent Application Laid-Open Publication No. 05-337073 discloses a technique including a trocar with a fiber probe for guide light incorporated in the trocar, the fiber probe being provided for guiding laser light emitted from a laser light source apparatus, and a grasping forceps to be inserted into a body cavity via the trocar, in which laser light emitted from the trocar through the fiber probe for guide light is used as light for guiding a distal end portion of the grasping forceps inserted through an insertion passage of the trocar into a view field of a laparoscope.

Also, when treatment of an examined region inside a body cavity is performed using an optical fiber and a medical device in combination, it is desirable to prevent a force generated in response to operation of the medical device by a surgeon from being exerted as a factor of mechanical damage (such as breakage) of the optical fiber.

According to the configuration of the trocar disclosed in Japanese Patent Application Laid-Open Publication No. 05-337073, the fiber probe for guide light is provided at a position that is different from that of the insertion passage through which the grasping forceps is inserted, to prevent, as much as possible, a force generated in response to operation of the grasping forceps from being exerted as a factor of mechanical damage of the fiber probe for the guide light.

However, according to the configuration disclosed in Japanese Patent Application Laid-Open Publication No. 05-337073, a configuration for preventing occurrence of mechanical damage of the fiber probe for guide light is incorporated in the trocar, which causes the problem of a low degree of freedom in configuration for performing treatment of an examined region inside a body cavity using the fiber probe for guide light and the grasping forceps in combination. Therefore, according to the configuration disclosed in Japanese Patent Application Laid-Open Publication No. 05-337073, there arises an issue according to the aforementioned problem of a low degree of freedom in configuration for performing treatment of an examined region inside a body cavity using an optical fiber and a medical device in combination.

The present invention has been made in view of the aforementioned circumstances, and an object of the present invention is to provide a medical sheath and a medical appliance configured to, while enhancing a degree of freedom in configuration for performing treatment of an examined region inside a body cavity using an optical fiber and a medical device in combination, enable prevention of occurrence of mechanical damage of the optical fiber.

### Disclosure of Invention

### Means for Solving the Problem

A medical sheath according to an aspect of the present invention is a medical sheath to be used in combination with a medical device including an insertion portion that can be inserted into a body cavity of a subject via a trocar when treatment of an examined region present in the body cavity of the subject is performed using the medical device, the medical sheath including: a sheath body formed in a shape that can be inserted to and removed from a through hole through which the insertion portion is inserted in the trocar; a channel provided in the sheath body, the channel being a conduit formed so as to have a shape that enables insertion and removal of the insertion portion; and at least one optical fiber provided in the sheath body, the optical fiber being provided for guiding illuminating light to be applied to the examined region and return light emitted from the examined region upon application of the illuminating light.

A medical appliance according to an aspect of the present invention is a medical appliance including: a medical device including an insertion portion that can be inserted into a body cavity of a subject via a trocar; a sheath body to be used in combination with the medical device when treatment of an examined region present in the body cavity of the subject is performed using the medical device, the sheath body being formed in a shape that can be inserted to and removed from a through hole through which the insertion portion is inserted in the trocar; a channel provided in the sheath body, the channel being a conduit formed so as to have a shape that enables insertion and removal of the insertion portion; and an optical transmission section provided in the sheath body, the optical transmission section being provided for guiding at least one of illuminating light to be applied to the examined region and return light emitted from the examined region upon application of the illuminating light.

### Brief Description of the Drawings

Fig. 1 is a diagram illustrating a configuration of a major part of a medical system including a medical sheath according to an embodiment;
Fig. 2 is a cross-sectional view for describing a configuration of the medical sheath according to the embodiment;
Fig. 3 is a diagram illustrating an example of a state in which the medical sheath according to the embodiment is inserted from the proximal end side of a through hole of a trocar TR;
Fig. 4 is a diagram for describing a configuration of a medical sheath according to a modification of the embodiment;
Fig. 5 is a cross-sectional view for describing a configuration of a medical sheath according to a modification of the embodiment;
Fig. 6 is a cross-sectional view for describing a configuration of a medical sheath according to a modification of the embodiment;
Fig. 7 is a cross-sectional view for describing a configuration of a medical sheath according to a modification of the embodiment;
Fig. 8 is a cross-sectional view for describing a configuration of a medical sheath according to a modification of the embodiment; and
Fig. 9 is a diagram for describing a configuration of a medical sheath according to a modification of the embodiment.

### Best Mode for Carrying Out the Invention

An embodiment of the present invention will be described below with reference to the drawings.

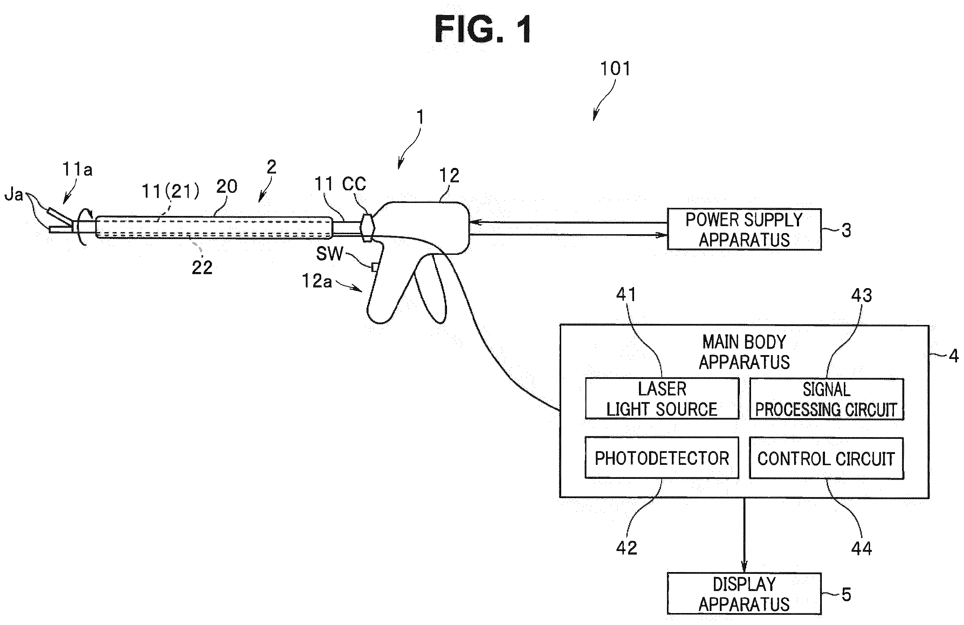

Figs. 1 to 9 relate to an embodiment of the present invention and modifications.

As illustrated in Fig. 1, a medical system 101 includes a medical device 1, a medical sheath 2, a power supply apparatus 3, a main body apparatus 4 and a display apparatus 5. Fig. 1 is a diagram illustrating a configuration of a major part of a medical system including a medical sheath according to an embodiment.

The medical device 1 is configured, for example, as a treatment instrument via which treatment can be performed by insertion of the treatment instrument into a body cavity of an examinee and application of energy according to power supplied from the power supply apparatus 3 to an examined region such as a living tissue present inside the body cavity of the examinee. More specifically, the medical device 1 includes, for example, a bipolar-type electric scalpel or a high-frequency treatment instrument. Also, the medical device 1 includes, for example, an insertion portion 11 and an operation portion 12 as illustrated in Fig. 1.

The insertion portion 11 is formed so as to have an outer appearance shape that can be inserted into a body cavity of a subject via a trocar to be installed in a body wall of the subject such as, for example, an elongated cylindrical shape. Also, the insertion portion 11 includes a treatment portion 11a provided on the distal end side, the treatment portion 11a being provided for applying energy according to power supplied from the power supply apparatus 3. Also, the insertion portion 11 is connected to the operation portion 12 via a connection connector CC provided on the proximal end side.

Here, the connection connector CC in the present embodiment has a structure for connecting the insertion portion 11 to the operation portion 12 in such a manner that the insertion portion 11 is infinitely rotatable relative to the operation portion 12. In other words, the insertion portion 11 in the present embodiment is configured so that, upon the insertion portion 11 being connected to the operation portion 12 via the connection connector CC, the insertion portion 11 can infinitely rotate with a longitudinal direction as a rotation axis.

As illustrated in Fig. 1, the treatment portion 11a includes, for example, a pair of jaws Ja configured to be opened/closed in response to operation of the operation portion 12, and is configured so as to be able to apply energy to, e.g., a living tissue grasped by the pair of jaws Ja.

The operation portion 12 is connected to the insertion portion 11 via the connection connector CC. Also, the operation portion 12 includes a handle portion 12a via which operation relating to opening/closing of the pair of jaws Ja of the treatment portion 11a can be performed. Also, at the handle portion 12a, a switch SW via which operation for turning on or off power supply from the power supply apparatus 3 can be performed is provided.

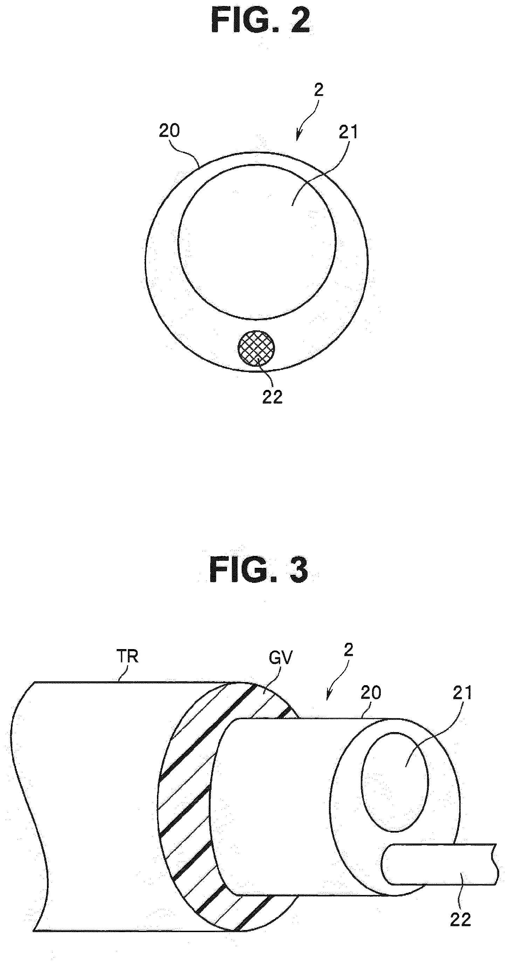

The medical sheath 2 includes, for example, a sheath body 20 formed in an outer appearance shape that can be inserted to and removed from a through hole that is a hole, through which the insertion portion 11 is inserted, in a trocar to be installed in a body wall of a subject, such as an elongated cylindrical shape. Also, the medical sheath 2 has a cross-sectional shape illustrated as an example in Fig. 2 in a cross-section perpendicular to a longitudinal direction of the sheath body 20. Also, as illustrated in Fig. 2, in the sheath body 20 of the medical sheath 2, a treatment instrument channel 21 and an optical fiber 22 are provided. Fig. 2 is a cross-sectional view for describing the medical sheath according to the embodiment.

The sheath body 20 includes a low thermal conductivity material that can protect the optical fiber 22 against heat generated upon energy application in the treatment portion 11a, such as, for example, silicone or fluorine resin.

Inside the sheath body 20, for example, as illustrated in Figs. 1 and 2, the treatment instrument channel 21 configured to enable the insertion portion 11 inserted from the proximal end side to be disposed so as to be inserted through the treatment instrument channel 21 and also enables the treatment portion 11a to project from the distal end side is formed. Also, inside the sheath body 20, for example, as illustrated in Figs. 1 and 2, one optical fiber 22 for guiding each of illuminating light supplied from the main body apparatus 4 and return light generated upon application of the illuminating light is disposed so as to be inserted through the sheath body 20.

The treatment instrument channel 21, which is a conduit formed so as to have a shape that enables insertion and removal of the insertion portion 11, is provided along the longitudinal direction of the sheath body 20. Also, the treatment instrument channel 21 is formed so as to have an inner diameter that is substantially equal to an outer diameter of the insertion portion 11 having, for example, a cylindrical shape, and is formed so as to have a center axis at a position deviated from a center axis of the sheath body 20. Also, an inner peripheral face of the treatment instrument channel 21 is subjected to a process for enhancing operability of the insertion portion 11 inserted through the treatment instrument channel 21 such as, for example, fluorine resin coating.

The configuration of the treatment instrument channel 21 as above enables elimination of a gap between an outer peripheral face of the insertion portion 11 and the inner peripheral face of the treatment instrument channel 21 when the insertion portion 11 is inserted through the treatment instrument channel 21. Also, the configuration of the treatment instrument channel 21 as above enables the insertion portion 11 inserted through the treatment instrument channel 21 to be smoothly advanced/retracted and rotated.

The optical fiber 22 is disposed so as to form no gap between the optical fiber 22 and the sheath body 20, and is provided along the longitudinal direction of the sheath body 20.

A proximal end portion of the optical fiber 22 is provided so as to extend out from the proximal end side of the sheath body 20, and is configured so as to be detachably connected to the main body apparatus 4. Also, a distal end portion of the optical fiber 22 is disposed on the distal end side of the sheath body 20. Also, in the vicinity of an end face of the distal end portion of the optical fiber 22, a condenser lens (not illustrated) for collecting illuminating light to be emitted through the end face, and collecting return light generated upon application of the illuminating light and making the light enter the end face is provided.

The power supply apparatus 3 includes, for example, a high-frequency power supply, and is configured so as to, if the switch SW in the handle portion 12a is on, perform power supply to the medical device 1, and if the switch SW in the handle portion 12a is off, stop the power supply to the medical device 1.

The main body apparatus 4 includes a laser light source 41, a photodetector 42, a signal processing circuit 43 and a control circuit 44.

The laser light source 41 includes, for example, a laser diode, and is configured so as to generate laser light as illuminating light to be applied to an examined region, which is to be treated by the medical device 1, and supply the laser light to the optical fiber 22 connected to the main body apparatus 4.

The photodetector 42 includes, for example, an avalanche photodiode, and is configured so as to detect return light emitted through the optical fiber 22 connected to the main body apparatus 4, and generate and output a photodetection signal according to an intensity of the detected return light.

The signal processing circuit 43 is configured so as to perform, for example, predetermined signal processing on a photodetection signal outputted from the photodetector 42 to generate an image visualizing a state of an examined region to be treated by the medical device 1, and output the image.

The control circuit 44 is configured so as to perform, for example, control for adjusting an amount of laser light emitted from the laser light source 41 according to a signal level of a photodetection signal outputted from the photodetector 42, as control relating to operation of the respective sections of the main body apparatus 4.

The display apparatus 5 includes a liquid-crystal display or the like and is configured so as to, for example, display, e.g., an image outputted from the main body apparatus 4.

Subsequently, operation of the present embodiment will be described.

A user such as a surgeon connects the respective sections in the medical system 101 and turns on the power and then, for example, expands an abdominal cavity of an examinee using a pneumoperitoneum gas suppled from a pneumoperitoneum apparatus (not illustrated), and continuously inserts the medical sheath 2 from the proximal end side of a through hole of a trocar TR in a state in which the trocar TR is installed in an abdominal wall of the examinee. Then, with such operation by the user, for example, the sheath body 20 is disposed so as to be inserted through the through hole in a state in which the sheath body 20 is held by a gas leakage prevention rubber valve GV provided on the proximal end side of the through hole of the trocar TR (see Fig. 3). Fig. 3 is a diagram illustrating an example of a state in which the medical sheath according to the embodiment is inserted from the proximal end side of the through hole of the trocar TR.

The user disposes the medical sheath 2 in the trocar TR so as to be inserted through the trocar TR in a state in which the insertion portion 11 of the medical device 1 is inserted from the proximal end side of the treatment instrument channel 21 of the medical sheath 2. After the insertion portion 11 is disposed inside the treatment instrument channel 21 so as to be inserted through the treatment instrument channel 21, treatment of an examined region present inside an abdominal cavity of an examinee is performed by turning the switch SW on in a state in which the treatment portion 11a projects from the distal end side of the treatment instrument channel 21.

Here, in the present embodiment, the sheath body 20 is held by the rubber valve GV provided on the proximal end side of the through hole of the trocar TR, and the inner peripheral face of the treatment instrument channel 21 is subjected to a process for enhancing operability of the insertion portion 11 inserted through the treatment instrument channel 21, and thus, a frictional force exerted on a surface of contact between the sheath body 20 and the rubber valve GV is relatively larger than a frictional force exerted on a surface of contact between the insertion portion 11 and the treatment instrument channel 21.

Therefore, according to the present embodiment, for example, even if operation to rotate the insertion portion 11 with the longitudinal direction as a rotation axis is performed during treatment of an examined region inside an abdominal cavity of an examinee being performed in a state in which the insertion portion 11 is inserted through the treatment instrument channel 21, rotation of the sheath body 20 that may occur following the rotation of the insertion portion 11 can be suppressed, which enables prevention of occurrence of mechanical damage of the optical fiber 22. Also, according to the present embodiment, for example, the gas leakage prevention rubber valve GV is attached to a trocar TR desired by a user and the medical sheath 2 is disposed so as to be inserted through the trocar TR, which enables treatment of an examined region inside a body cavity to be performed while using the optical fiber 22 and the medical device 1 in combination. In other words, according to the present embodiment, while a degree of freedom in configuration for performing treatment of an examined region inside a body cavity using an optical fiber and a medical device in combination is enhanced, occurrence of mechanical damage of the optical fiber can be prevented.

Here, the medical sheath 2 according to the present embodiment may be configured in such a manner that, for example, a gas leakage prevention rubber valve having a function that is similar to that of the rubber valve GV is provided on the proximal end side of the treatment instrument channel 21.

Also, according to the present embodiment, the optical fiber 22 is not limited to an optical fiber having a configuration that can guide both illuminating light and return light, and the optical fiber 22 may have a configuration that can guide only either illuminating light or return light. In other words, the optical fiber 22 in the present embodiment may only have a configuration for guiding at least one of illuminating light supplied from the main body apparatus 4 and return light generated upon application of the illuminating light.

Also, according to the present embodiment, as long as a frictional force exerted on the surface of contact between the sheath body 20 and the trocar TR is relatively larger than a frictional force exerted on the surface of contact between the insertion portion 11 and the treatment instrument channel 21, the inner peripheral face of the treatment instrument channel 21 may be subjected to no process (e.g., coating) for enhancing operability of the insertion portion 11 inserted through the treatment instrument channel 21.

Also, according to the present embodiment, for example, it is possible that: the optical fiber 22 is divided in two parts that are a part extending out from the proximal end side of the sheath body 20 and a part incorporated in the sheath body 20; and the two parts are detachably attachable to each other.

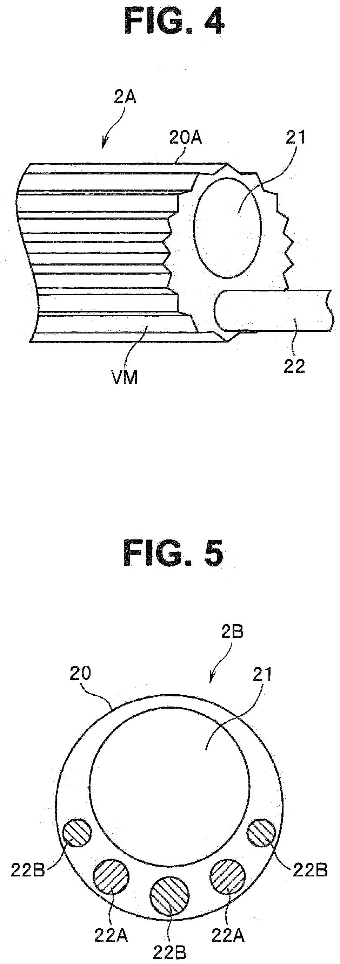

Also, the medical sheath 2 in the present embodiment may be, for example, a medical sheath configured as a medical sheath 2A including a sheath body 20A having an outer appearance shape such as illustrated in Fig. 4. Note that, hereinafter, for sake of simplicity, a detailed description of parts to which components described above can apply will be arbitrarily omitted. Fig. 4 is a diagram for describing a configuration of a medical sheath according to a modification of the embodiment.

The sheath body 20A includes a rotation suppressing structure in an outer peripheral face, the rotation suppressing structure being capable of suppressing rotation that can occur following rotation of an insertion portion 11 inserted through a treatment instrument channel 21 in a state in which the sheath body 20A is inserted through a trocar TR. More specifically, in the outer peripheral face of the sheath body 20A, for example, a plurality of V-shape grooves VM formed so as to extend in a direction parallel to a longitudinal direction of the sheath body 20A are provided as the aforementioned rotation suppressing structure. Here, the sheath body 20A has a configuration that is substantially similar to the configuration of the sheath body 20 in the parts other than the aforementioned plurality of grooves VM.

The medical sheath 2A including the sheath body 20A described above enables further suppression of rotation that may occur following rotation of the insertion portion 11 inserted through the treatment instrument channel 21.

Here, the grooves formed in the outer peripheral face of the sheath body 20A may have a shape other than a V shape. Also, the grooves formed in the outer peripheral face of the sheath body 20A may be formed over an entire length in the longitudinal direction or may be formed in only a part that may be in contact with a rubber valve GV of a trocar TR.

Also, the medical sheath 2 according to the present embodiment is not limited to a medical sheath having a cross-sectional shape such as illustrated in Fig. 2, and may be, for example, a medical sheath configured as a cylindrical medical sheath 2B having a cross-sectional shape such as illustrated in Fig. 5. Fig. 5 is a cross-sectional view for describing a configuration of a medical sheath according to a modification of the embodiment.

As illustrated in Fig. 5, the medical sheath 2B includes, for example, a plurality of optical fibers 22A for guiding illuminating light supplied from a main body apparatus 4 and a plurality of optical fibers 22B for guiding return light generated upon application of the illuminating light, which are each provided in a sheath body 20, instead of the optical fiber 22 in the medical sheath 2.

As illustrated in Fig. 5, the plurality of optical fibers 22A and 22B are, for example, disposed in the vicinity of the treatment instrument channel 21 in such a manner that the optical fibers 22A and 22B are alternately arranged in a rough fan-like shape.

A proximal end portion of each of the plurality of optical fibers 22A and 22B has a configuration that is similar to the configuration in the optical fiber 22, that is, is provided so as to extend out from the proximal end side of the sheath body 20 and is configured so as to be detachably connected to a main body apparatus 4. Also, a distal end portion of each of the plurality of optical fibers 22A and 22B has a configuration that is similar to the configuration in the optical fiber 22, that is, is provided on the distal end side of the sheath body 20. Also, in the vicinity of each of end faces of the distal end portions of the plurality of optical fibers 22A, a condenser lens (not illustrated) for collecting illuminating light to be emitted through the relevant end face is provided. Also, in the vicinity of each of end faces of the distal end portions of the plurality of optical fibers 22B, a condenser lens (not illustrated) for collecting return light generated upon application of illuminating light and making the return light enter the relevant end face is provided.

According to the medical sheath 2B having the above-described configuration, for example, even if jaws Ja in an opened state may be a factor interrupting an optical path, illuminating light can be applied to an examined region from at least one of the plurality of optical fibers 22A, and return light emitted from the examined region can be received by at least one of the plurality of optical fibers 22B. Also, the medical sheath 2B having the above-described configuration enables application of an amount of illuminating light, the amount being larger than an amount of illuminating light in the medical sheath 2, to an examined region and also enables enhancement in sensitivity of reception of return light emitted from the examined region relative to the medical sheath 2. Also, the medical sheath 2B having the above-described configuration enables reduction in outer diameter relative to a medical sheath 2C, which will be described later.

Also, according to the present embodiment, where the medical system 101 includes the medical sheath 2B and optical adjustment is made so that illuminating light emitted from a laser light source 41 is applied to respective positions that are mutually substantially the same through the plurality of optical fibers 22A, supply of the illuminating light is not limited to simultaneous supply of the illuminating light to the plurality of optical fibers 22A, and, for example, switching control for sequentially switching an optical fiber 22A that is a destination of supply of the illuminating light from one optical fiber to another optical fiber may be performed.

More specifically, the control circuit 44 may be configured so as to, for example, if a non-illustrated optical switch such as a MEMS optical switch is provided on an optical path from the laser light source 41 to each of end faces of the proximal end portions of the plurality of optical fibers 22A is provided, perform control to sequentially switch the end face of one optical fiber 22A that is a destination of emission of light through the relevant optical switch in a predetermined cycle as the aforementioned switching control. Here, the aforementioned predetermined cycle may be set to be, for example, a period of time that is longer than a period of time of sampling when FFT (fast Fourier transformation) is performed as signal processing in the signal processing circuit 43.

According to the switching control described above, illuminating light emitted from the laser light source 41 can be supplied to a single optical fiber 22A in a concentrated manner, and thus, a density of illuminating light applied to an examined region through the single optical fiber 22A can be increased as much as possible, and as a result, a received light amount when return light emitted from the examined region is received by the plurality of optical fibers 22B can be increased as much as possible.

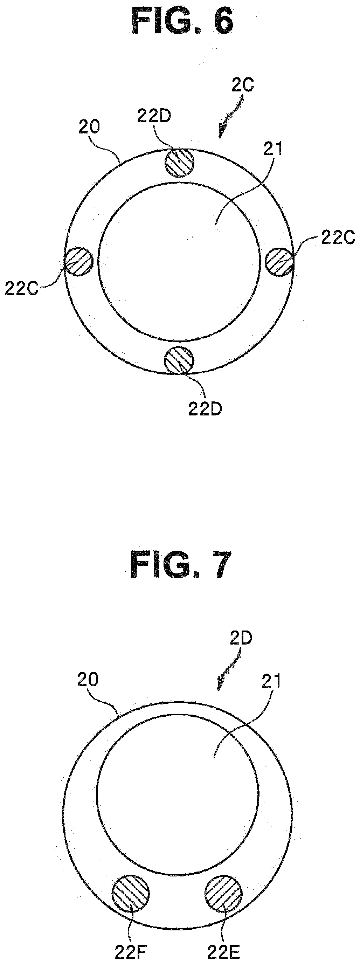

Also, the medical sheath 2 according to the present embodiment is not limited to a medical sheath having a cross-sectional shape such as illustrated as an example in Fig. 2, and may be, for example, a medical sheath configured as a cylindrical medical sheath 2C having a cross-sectional shape such as illustrated in Fig. 6. Fig. 6 is a cross-sectional view for describing a configuration of a medical sheath according to a modification of the embodiment.

As illustrated in Fig. 6, a treatment instrument channel 21 of the medical sheath 2C is formed, for example, so as to have a center axis at a position that is the same as a position of a center axis of a cylindrical sheath body 20. Also, as illustrated in Fig. 6, the medical sheath 2C includes, for example, a plurality of optical fibers 22C for guiding illuminating light supplied from a main body apparatus 4 and a plurality of optical fibers 22D for guiding return light generated upon application of the illuminating light, which are each provided in the sheath body 20, instead of the optical fiber 22 in the medical sheath 2.

As illustrated in Fig. 6, the plurality of optical fibers 22C and 22D are, for example, disposed in a periphery of the treatment instrument channel 21 in such a manner that the optical fibers 22C and 22D are alternately arranged at a substantially equal interval.

A proximal end portion of each of the plurality of optical fibers 22C and 22D has a configuration that is similar to the configuration in the optical fiber 22, that is, is provided so as to extend out from the proximal end side of the sheath body 20 and configured so as to be detachably connected to the main body apparatus 4. Also, a distal end portion of each of the plurality of optical fibers 22C and 22D has a configuration that is similar to the configuration in the optical fiber 22, that is, is disposed on the distal end side of the sheath body 20. Also, in the vicinity of each of end faces of the distal end portions of the plurality of optical fibers 22C, a condenser lens (not illustrated) for collecting illuminating light to be emitted through the relevant end face is provided. Also, in the vicinity of each of end faces of the distal end portions of the plurality of optical fibers 22D, a condenser lens (not illustrated) for collecting return light generated upon application of illuminating light and making the return light enter the relevant end face is provided.

According to the medical sheath 2C having the above-described configuration, for example, even if jaws Ja in an opened state may be a factor interrupting an optical path, illuminating light can be applied to an examined region from at least one of the plurality of optical fibers 22C, and return light emitted from the examined region can be received by at least one of the plurality of optical fibers 22D. Also, the medical sheath 2C having the above-described configuration enables application of an amount of illuminating light, the amount being larger than an amount of illuminating light in the medical sheath 2, to an examined region and also enables enhancement in sensitivity of reception of return light emitted from the examined region relative to the medical sheath 2.

Also, the medical sheath 2 according to the present embodiment is not limited to a medical sheath having a cross-sectional shape such as illustrated as an example in Fig. 2, and may be, for example, a medical sheath configured as a cylindrical medical sheath 2D having a cross-sectional shape such as illustrated in Fig. 7. Fig. 7 is a cross-sectional view for describing a configuration of a medical sheath according to a modification of the embodiment.

As illustrated in Fig. 7, the medical sheath 2D includes one optical fiber 22E for guiding illuminating light supplied from a main body apparatus 4 and one optical fiber 22F for guiding return light generated upon application of the illuminating light, which are each provided in a sheath body 20, instead of the optical fiber 22 in the medical sheath 2.

A proximal end portion of each of the optical fibers 22E and 22F has a configuration that is similar to the configuration in the optical fiber 22, that is, is provided so as to extend out from the proximal end side of the sheath body 20 and is configured so as to be detachably connected to the main body apparatus 4. Also, a distal end portion of each of the optical fibers 22E and 22F has a configuration that is similar to the configuration in the optical fiber 22, that is, is provided on the distal end side of the sheath body 20. Also, in the vicinity of an end face of the distal end portion of the optical fiber 22E, a condenser lens (not illustrated) for collecting illuminating light to be emitted through the end face is provided. Also, in the vicinity of an end face of the distal end portion of the optical fiber 22F, a condenser lens (not illustrated) for collecting return light generated upon application of illuminating light and making the return light enter the end face is provided.

Here, in a medical system 101 including the medical sheath 2D, for example, as a result of jaws Ja in an opened state operating as an obstacle interrupting an optical path, at least either one of a situation in which illuminating light emitted through the optical fiber 22E does not reach an examined region and a situation in which return light emitted from the examined region does not enter the optical fiber 22F may occur. Therefore, in the medical system 101 including the medical sheath 2D, for example, a control circuit 44 in the main body apparatus 4 performs control to, if it is detected that a signal level of a photodetection signal outputted from a photodetector 42 is 0 or less than a predetermined threshold value, inform a user of interruption of the optical path by the jaws Ja in an opened state. Here, in the present embodiment, in response to the control relating the informing by the control circuit 44, for example, a predetermined warning sound may be issued from a non-illustrated speaker provided in the main body apparatus 4, a non-illustrated light emitting element provided in the main body apparatus 4 may blink in a predetermined pattern or a predetermined icon or the like may be displayed on a display apparatus 5.

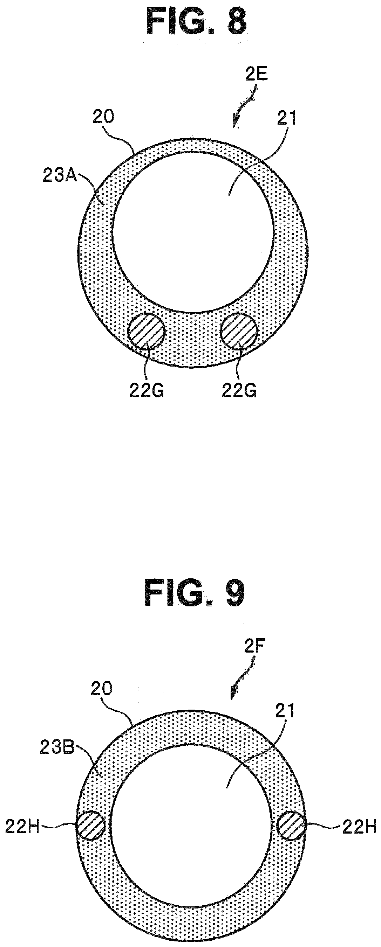

Also, the medical sheath 2 according to the present embodiment is not limited to a medical sheath having a cross-sectional shape such as illustrated as an example in Fig. 2, and may be, for example, a medical sheath configured as a cylindrical medical sheath 2E having a cross-sectional shape such as illustrated in Fig. 8. Fig. 8 is a cross-sectional view for describing a configuration of a medical sheath according to a modification of the embodiment.

As illustrated in Fig. 8, the medical sheath 2E includes two optical fibers 22G for guiding illuminating light supplied from a main body apparatus 4, which are provided in a sheath body 20, instead of the optical fiber 22 in the medical sheath 2. Also, in a part of an inner portion of the sheath body 20 other than a treatment instrument channel 21 and the optical fibers 22G, the part corresponding to the dotted part in Fig. 8, an optical fiber bundle 23A including a plurality of optical fibers for guiding return light generated upon application of illuminating light is charged with no gap.

The medical sheath 2E having the above-described configuration enables enhancement in sensitivity of reception of return light generated upon application of illuminating light relative to the medical sheath 2.

Also, the medical sheath 2 according to the present embodiment is not limited to a medical sheath having a cross-sectional shape such as illustrated as an example in Fig. 2, and may be, for example, a medical sheath configured as a cylindrical medical sheath 2F having a cross-sectional shape such as illustrated in Fig. 9. Fig. 9 is a cross-sectional view for describing a configuration of a medical sheath according to a modification of the embodiment.

As illustrated in Fig. 9, a treatment instrument channel 21 of the medical sheath 2F is formed, for example, so as to have a center axis at a position that is the same as a position of a center axis of a cylindrical sheath body 20. Also, as illustrated in Fig. 9, the medical sheath 2F includes, for example, two optical fibers 22H for guiding illuminating light supplied from a main body apparatus 4, which are provided in the sheath body 20, instead of the optical fiber 22 in the medical sheath 2. Also, in a part of an inner portion of the sheath body 20 other than the treatment instrument channel 21 and the optical fibers 22H, the part corresponding to the dotted part in Fig. 9, an optical fiber bundle 23B including a plurality of optical fibers for guiding return light generated upon application of illuminating light is charged with no gap.

The medical sheath 2F having the above-described configuration enables enhancement in sensitivity of reception of return light generated upon application of illuminating light relative to the medical sheath 2.

Here, according to the present embodiment, for example, a medical sheath may be configured so that the medical sheath itself serves as a light guiding path, by forming the sheath body 20 using a transparent member that can guide both illuminating light and return light and is resistant to heat generated from the treatment portion 11a, instead of provision of a light guiding member such as the optical fiber 22 in the sheath body 20.

Also, according to the present embodiment, for example, an optical fiber 22 may be disposed in a part corresponding to or substantially corresponding to a center axis in the longitudinal direction of the insertion portion 11, so as to be inserted through the insertion portion 11, instead of provision of the optical fiber 22 in the sheath body 20.

The present invention is not limited to the embodiment and modifications described above, and it should be understood that various alterations and applications are possible without departing from the spirit of the present invention.

The present application is filed claiming priority from Japanese Patent Application No. 2015-162859 filed in Japan on August 20, 2015, the disclosure of which is incorporated in the description, the claims and the drawings of the present application by reference.

## Claims

1. A medical sheath to be used in combination with a medical device including an insertion portion that can be inserted into a body cavity of a subject via a trocar when treatment of an examined region present in the body cavity of the subject is performed using the medical device, the medical sheath comprising:
a sheath body formed in a shape that can be inserted to and removed from a through hole through which the insertion portion is inserted in the trocar;
a channel provided in the sheath body, the channel being a conduit formed so as to have a shape that enables insertion and removal of the insertion portion; and
an optical transmission section provided in the sheath body, the optical transmission section being provided for guiding at least one of illuminating light to be applied to the examined region and return light emitted from the examined region upon application of the illuminating light.

2. The medical sheath according to claim 1, wherein the sheath body includes a rotation suppressing structure provided in an outer peripheral face, the rotation suppressing structure being capable of suppressing rotation that can occur following rotation of the insertion portion inserted through the channel in a state in which the sheath body is inserted through the trocar.

3. The medical sheath according to claim 2, wherein the rotation suppressing structure includes a plurality of grooves formed so as to extend in a direction parallel to a longitudinal direction of the sheath body.

4. The medical sheath according to claim 1, wherein the channel is formed so as to have an inner diameter that is substantially equal to an outer diameter of the insertion portion having a cylindrical shape.

5. A medical appliance comprising:
a medical device including an insertion portion that can be inserted into a body cavity of a subject via a trocar;
a sheath body to be used in combination with the medical device when treatment of an examined region present in the body cavity of the subject is performed using the medical device, the sheath body being formed in a shape that can be inserted to and removed from a through hole through which the insertion portion is inserted in the trocar;
a channel provided in the sheath body, the channel being a conduit formed so as to have a shape that enables insertion and removal of the insertion portion; and
an optical transmission section provided in the sheath body, the optical transmission section being provided for guiding at least one of illuminating light to be applied to the examined region and return light emitted from the examined region upon application of the illuminating light.

6. The medical appliance according to claim 5, further comprising a control section configured to, if a plurality of optical fibers for guiding the illuminating light emitted from a light source section are provided in the optical transmission section, perform switching control for sequentially switching an optical fiber that is a destination of supply of the illuminating light emitted from the light source section from one optical fiber to another optical fiber.
